# EUROPEAN PATENT SPECIFICATION

(11) **EP 4 398 895 B1**
(45) Date of publication and mention of the grant of the patent: **07.05.2025**
(21) Application number: 23748476.1
(22) Date of filing: 25.07.2023
(51) Int. Cl.: A61K 31/198, A61K 31/381, A61P 29/00

(54) **ERDOSTEINE, SALTS, ENANTIOMERS OR METABOLITES THEREOF FOR USE IN THE TREATMENT OF NOCIPLASTIC AND NEUROPATHIC PAIN STATES**
ERDOSTEIN, SALZE, ENANTIOMERE ODER METABOLITE DAVON ZUR VERWENDUNG BEI DER BEHANDLUNG VON NOZIPLASTISCHEN UND NEUROPATHISCHEN SCHMERZZUSTÄNDEN
ERDOSTEINE, SELS, ÉNANTIOMÈRES OU LEURS MÉTABOLITES POUR UTILISATION DANS LE TRAITEMENT DES ÉTATS DOULOUREUX NOCIPLASTIQUES ET NEUROPATHIQUES

(30) Priority: 01.08.2022 EP 22188031
(43) Date of publication of application: 17.07.2024
(73) Proprietor: Edmond Pharma Srl, 20037 Paderno Dugnano (MI) (IT)
(72) Inventor: GOVONI, Stefano, 27100 PAVIA (IT); ALLEGRI, Massimo, 24121 BERGAMO (IT); MARCHESI, Nicoletta, 27040 PINAROLO PO (PV) (IT); PASCALE, Alessia, 27100 PAVIA (IT)
(74) Representative: Bianchetti & Minoja with Trevisan & Cuonzo IPS SRL
(86) International application number: PCT/EP2023/070563
(87) International publication number: WO 2024/028157

(56) References cited:
- WO-A1-2020/139240
- XI YANG ET AL: "Protective effects of Erdosteine on interleukin-1[beta]-stimulated inflammation via inhibiting the activation of MAPK, NF-[kappa]B, and Wnt/[beta]-catenin signaling pathways in rat osteoarthritis", EUROPEAN JOURNAL OF PHARMACOLOGY, ELSEVIER SCIENCE, NL, vol. 873, 17 January 2020 (2020-01-17), XP086071749, ISSN: 0014-2999, [retrieved on 20200117], DOI: 10.1016/J.EJPHAR.2020.172925
- CAZZOLA MARIO ET AL: "Multifaceted Beneficial Effects of Erdosteine: More than a Mucolytic Agent", vol. 80, no. 17, 6 October 2020 (2020-10-06), NZ, pages 1799 - 1809, XP093009927, ISSN: 0012-6667, Retrieved from the Internet <URL:https://link.springer.com/article/10.1007/s40265-020-01412-x/fulltext.html> DOI: 10.1007/s40265-020-01412-x
- MIYAKE K ET AL: "THE EFFECT OF ERDOSTEINE AND ITS ACTIVE METABOLITE ON REACTIVE OXYGEN SPECIES PRODUCTION BY INFLAMMATORY CELLS", INFLAMMATION RESEARCH, BIRKHAEUSER VERSLAG , BASEL, CH, vol. 48, no. 4, 1 April 1999 (1999-04-01), pages 205 - 209, XP000992518, ISSN: 1023-3830, DOI: 10.1007/S000110050447
- FITZCHARLES MARY-ANN ET AL: "Nociplastic pain: towards an understanding of prevalent pain conditions", THE LANCET, ELSEVIER, AMSTERDAM, NL, vol. 397, no. 10289, 29 May 2021 (2021-05-29), pages 2098 - 2110, XP086583181, ISSN: 0140-6736, DOI: 10.1016/S0140-6736(21)00392-5
- HIROSE MUNETAKA ET AL: "NGF/TrkA Signaling as a Therapeutic Target for Pain", PAIN PRACTICE, vol. 16, no. 2, 27 August 2015 (2015-08-27), US, pages 175 - 182, XP093047842, ISSN: 1530-7085, DOI: 10.1111/papr.12342

## Description

The present invention concerns erdosteine, salts, enantiomers or its metabolite thereof, for use in the treatment of nociplastic and neuropathic pain states.

### Background of the invention

Several lines of evidence point to NGF and its signaling pathway through TrkA receptor as an important player in mechanisms sustaining various types of activities that, according to the context, may be considered either favorable, for example those promoting neuronal survival, or unfavorable (Oo WM., Hunter JD. et al., 2021).

It has also been found that mutations in the NGF gene cause the painlessness disease Hereditary Sensory and Autonomic Neuropathy type V (Testa GM. et al., 2021; Denk F. et al., 2021, Khan N., Smith TM., 2015). Hence, NGF/TrkA signaling is currently considered a potential target for pain therapy (Hirose M. et al., 2016). Furthermore, TrKA inhibition could be really helpful not only in neuropathic pain but also in nociplastic pain (definition from International Association for the Study of Pain https://www.iasp-pain.org/resources/terminology/?ItemNumber=1698, Aydede M., Shriver A et al., 2018) where central mechanisms who maintain/originate this kind of pain disease take place. In fact, nociplastic pain is a new clinical entity that could be driven with specific drugs designed to modulate this new disease with new potential targets (Pergolizzi JV Jr. et al., 2021).

The potential clinical applications of TrkA inhibition to treat pain are really well-established (Chang DS. et al., 2016; Yan W. et al., 2019). Previous studies have identified that the interaction between NGF and TrkA can be impaired by reducing thiolic compounds (Kamata H. et al., 2005). Furthermore, the target for this inhibition is not the nociceptive pain but other clinical entities like nociplastic pain or neuropathic pain.

An anti-NGF antibody capable of inhibiting the binding of NGF to TrkA has been proposed for the treatment or prevention of chronic pain (WO 2006/131951) but unfortunately a complete inhibition of NGF can cause severe side effects suggesting that a reversible modulation could be more effective.

Xi Yang et al.; European Journal of Pharmacology, vol.873, 2020, 172925 teaches that erdosteine exerts anti-inflammatory effects on IL-1beta-induced inflammatory response by reducing the expression of COX-2.

A small molecule, administered systemically, having the same target with higher safety profile, as erdosteine, accordingly to its present clinical use, would be obviously highly desirable to treat nociplastic pain and neuropathic pain.

### Description of the invention

We have now found that erdosteine, its salts, enantiomers and its active metabolite Met-1 inhibit TrkA activation by NGF and are therefore useful for the treatment of nociplastic or neuropathic pain.

Erdosteine, (2-[N-3-(2-oxotetrahydrothienyl)]acetamido)-thioglycolic acid) of formula 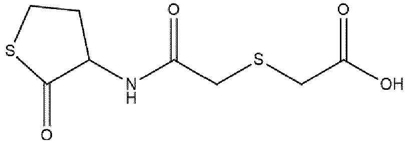 was first disclosed in FR 2,502,153 and US 4,411,909. Racemic R,S-Erdosteine is used in the therapy of chronic and acute respiratory disorders. It is converted following first-pass metabolism into its pharmacologically active metabolite Met-1 (Pharmacology 2006;77(3):150-4, 2006 Jul 3) of formula:

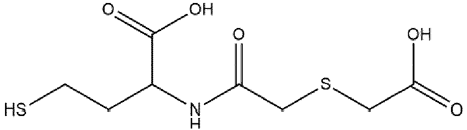

Erdosteine is currently registered as oral mucolytic drug, but also endowed with a well-established anti-oxidant and anti-inflammatory activity. Erdosteine exerts a protective role against lipid peroxidation (smokers, COPD patients) by increasing the availability of endogenous antioxidants, such as glutathione, in plasma and bronco-alveolar lavage fluid (BALF). In addition, erdosteine inhibits bacterial adhesion and enhances the antibiotic activity.

The term "Erdosteine" in the present description refers to any form thereof, including racemic form, single enantiomers, polymorphs and salts.

The invention is based on experiments showing that erdosteine and its metabolite Met-1 are able to inhibit TrkA activation by NGF, also suggesting that the effect is both time and concentration dependent, longer times of exposure (within the timeframe explored) driving higher inhibition rates. This pharmacological property is therefore of importance given the increasing relevance attributed to clinical applications of TrkA inhibition and pain relief (Chang DS. et al., 2016; Yan W. et al., 2019).

Erdosteine may be used according to the invention either in the racemic (R,S) form or in the form of one its enantiomers R or S, disclosed in EP 2 060 568, in whatever polymorphic form.

The metabolite Met-1 of erdosteine, disclosed inter alia in EP 974 353, has the following formula:

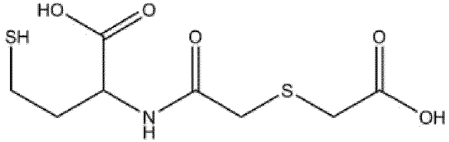

For the considered therapeutic use, erdosteine or its metabolite will be administered by any suitable route at dosages comparable to those already used in the clinical practice for the treatment of respiratory diseases, typically from 100 to 300 mg more times a day. The oral route is preferred but other routes conventionally used for analgesic drugs may be considered, e.g. the parenteral, transdermal, transmucosal routes. Examples of suitable dosage administration forms include capsules, tablets, controlled -release formulations, solutions, suspensions and the like.

Neuropathic and nociplastic pain may be successfully treated by administering erdosteine or its metabolite, both in acute and chronic phase.

The invention will now be illustrated in further details in the following experimental part and figures.

### Description of the figures

**Figure 1****:** MTT assay following exposure of SH-SY5Y cells for 90 minutes to erdosteine and its metabolite Met-1.
**Figure 2****:** MTT assay following exposure of SH-SY5Y cells for 24 hours to erdosteine and its metabolite Met-1.
**Figure 3****:** activation of the TrkA receptor by NGF following exposure of SH-SY5Y cells for 90 minutes to the metabolite of erdosteine Met-1.
**Figure 4****:** activation of the TrkA receptor by NGF following exposure of SH-SY5Y cells for 90 minutes to erdosteine or to its metabolite Met-1.
**Figure 5****:** activation of the TrkA receptor by NGF following exposure of SH-SY5Y cells for 90 minutes to the metabolite of erdosteine Met-1.
**Figure 6****:** activation of the TrkA receptor by NGF following exposure of SH-SY5Y cells for 90 minutes or 24 hours to 10 mM erdosteine.
**Figure 7****:** activation of the TrkA receptor by NGF following exposure of SH-SY5Y cells for 24 hours to concentrations of erdosteine equal or lower than 10 mM.
**Figure 8****:** effect of 24 hrs preincubation with 10 mM erdosteine and its R and S enantiomers on TrkA activation by NGF.
**Figure 9****:** effect of 24 hrs preincubation with different concentrations of erdosteine S enantiomer on TrkA activation by NGF.

### Detailed description of the invention

SH-SY5Y cell cultures were used to analyze the effect of erdosteine and its metabolite Met-1 on the activation of TrkA receptor by NGF.

In particular, the activation of TrkA has been induced using NGF 100 ng/ml (≅8 nM) for 10 min. The choice of time and concentration of NGF was based on previous experiences and preliminary experiments, which have indeed shown the ability of NGF to elicit in SH-SY5Y cells a several-fold increase of TrkA autophosphorylation. This is the first step in the chain of events elicited by NGF when it binds to the TrkA receptor (Marsh HN. et al., 2003) and therefore it is an index of TrkA activation.

The experiment is based on a commercially available ELISA assay evaluating the amount of phosphorylated TrkA (Pandre MK. et al., 2018) and used as indicated in the producer's protocol. The cell culture conditions were described in Marchesi N. et al., 2020.

Preliminary experiments were devoted to evaluate erdosteine and its metabolite *in vitro* toxicity (which may be expected at concentrations around and above 10 millimolar) and to investigate whether the time of exposure influenced this parameter. Accordingly, two time points were explored: short (90 min) and long (24 hrs). In general, longer times of exposure may sometime influence the chosen response to the test conditions/substances; it is therefore important in the starting exploratory protocols to explore an adequate time interval.

SH-SY5Y cells were pre-exposed to the test substances for the times indicated, then the medium was changed and a medium containing NGF was added; the time of exposure to NGF was 10 min as indicated by previous tests showing that this time was optimal for TrkA activation.

The cellular toxicity was evaluated by measuring the mitochondrial activity through the MTT assay (Marchesi N. et al., 2020).

The methods adopted, and described below, are standard methods as adapted according to the reported papers.

### Materials and methods

The following materials/substances were obtained by the indicated producers and methods applied as described.

### Cultured cells

Human neuroblastoma SH-SY5Y cells were obtained from ATCC (Manassas, VA) and cultured in T75 flasks in a humidified incubator at 37°C with 5% CO2. SH-SY5Y cells were grown in Eagle's minimum essential medium (EMEM) supplemented with 10% fetal bovine serum, 1% penicillin-streptomycin, L-glutamine (2 mM), non-essential amino acids (1 mM), and sodium pyruvate (1 mM).

In MTT experiments, the cells were exposed to 10, 20 and 30 mM erdosteine (Erdo; powder from Edmond Pharma); 10, 20 and 50 mM Metabolite-1 (Met-1; powder from Edmond Pharma).

For ELISA experiments, the cells were exposed to various concentrations of erdosteine and Met-1 for 90 minutes and 24 hours and then to 100 ng/ml NGF (rh beta-NGF; SRP3015,Sigma -Aldrich) for 10 minutes. The concentration and the time chosen for NGF were the best combination as derived from preliminary experiments using concentrations of NGF ranging from 0.5 to 1000 ng/ml and times from 5 to 60 min eliciting TrkA autophosphorylation response ranging from 2.5 to several folds the basal rate. All the experiments were performed under a laminar flow hood.

### MTT assay

Mitochondrial enzymatic activity was estimated by MTT [3-(4,5-dimethylthiazol-2-yl)-2,5-diphenyltetrazolium bromide] assay (Sigma-Aldrich). A cell suspension of 2×104 cells/mL SH-SY5Y cell line was seeded into 96-well plates.

Following each treatment of 90 minutes and 24 hours, 50 µL of MTT (concentration equal to 2.5 mg/mL) were added to each well. After incubation at 37° C for 3 hours, the purple formazan crystals were formed. The formed crystals were solubilized in dimethylsulfoxide (DMSO; Sigma-Aldrich). Specifically, after removing the MTT from the wells, 100 µL of DMSO were added in order to lyse the cellular and mitochondrial membranes, and solubilize the formazan crystals. After 10 minutes, absorbance values were measured at 595 nm using a Synergy HT microplate reader (BioTek Instruments) and the results expressed as % with respect to control.

### ELISA assay

The phospho-TrkA (Tyr674/675) protein levels in SH-SY5Y cells were estimated with a specific ELISA kit (Cell Signaling, #7212C.), according to the manufacturer's instructions.

PathScan^{®} Phospho-TrkA (Tyr674/675) Sandwich ELISA Kit is a solid phase sandwich enzyme-linked immunosorbent assay that detects transfected levels of Phospho-TrkA (Tyr674/675) protein. A TrkA Mouse mAb #4614 has been coated onto the microwells. After incubation with cell lysates, both phospho- and nonphospho-TrkA proteins are captured by the coated antibody. Following extensive washing to remove any unbound antibody/reagent, Phospho-TrkA (Tyr674/675) Antibody #4610 is added to detect phospho-TrkA protein. Anti-rabbit IgG, HRP-linked Antibody #7074 is then used to recognize the bound detection antibody. HRP substrate, TMB (3,3',5,5'-tetramethylbenzidine), is added to develop color. The magnitude of optical density is proportional to the quantity of Phospho-TrkA (Tyr674/675) protein.

The color development was stopped and the intensity of the color measured at 450 nm using a Synergy HT microplate reader (BioTek Instruments). The antibodies used in the kit were custom formulations specific for the kit.

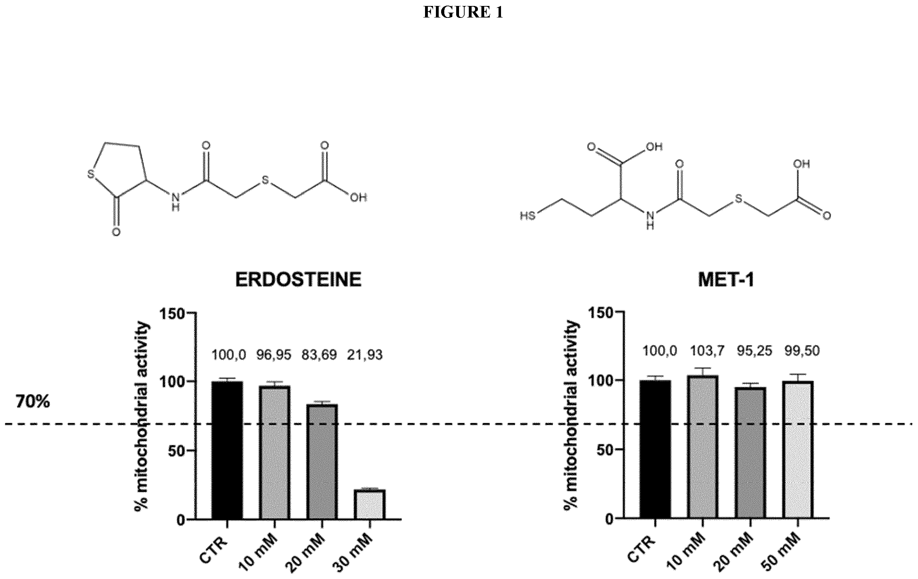

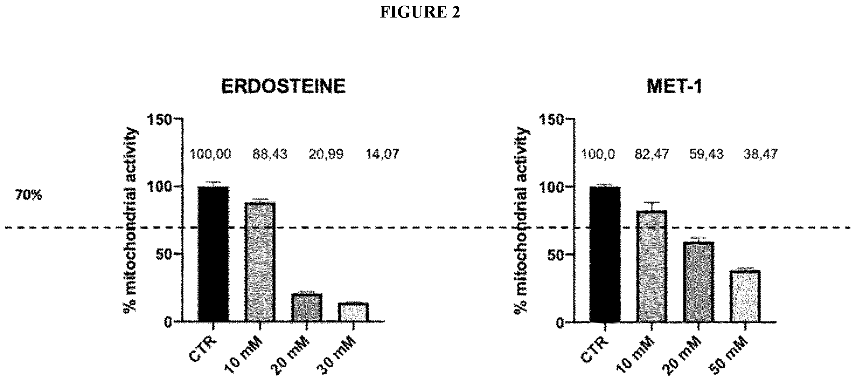

### 1. Tolerability of the test substances by SH-SY5Y cells after 90 minutes or 24 hrs of exposure (Figures 1 and 2).

The first assay performed was aimed to analyze the tolerability of SH-SY5Y cells to erdosteine and Met-1 using the MTT assay. A mitochondrial activity below 70% the control (CTR) level is considered an index of significant cellular toxicity (Srivastava GK. et al., 2018). For each test substance various concentrations were tested: from 10 to 50 mM. In the case of erdosteine the maximal concentration had to be limited to 30 mM because of solubility problems, while in the case of Met-1 concentrations up to 50 mM were tested. As it may be appreciated from figure 1, exposure of the cells to erdosteine 30 mM produced severe toxicity. Erdosteine 20 mM produced a 16.3% decrease in mitochondrial activity which is acceptable but indicates that cells start to suffer from the exposure to the substance, while erdosteine 10 mM was indistinguishable from control. The data on Met-1, the metabolite of erdosteine, indicate that all the concentrations used were perfectly tolerated at this time (90 min) of exposure. Bars at the top of the columns are standard errors in this as well as in all the other figures.

Next experiments were aimed to observe the tolerability to erdosteine and Met-1 at the same concentrations but after having exposed the cells for 24 hrs. Met-1 was soluble in water (164 mg/ml); erdosteine was soluble in MEM (11 mg/ml), warming 20 minutes at 37° allowing to expose the cells to the indicated final concentrations of the substances of interest.

As it may be appreciated from the graph in figure 2, at this time (24 hrs) erdosteine at both 20 and 30 mM produced a decrease of mitochondrial activity around and over 80%. Also Met-1 produced a significant change of mitochondrial activity at 24 hrs. at 20 and 50 mM concentrations. Met-1 10 mM produced a 17.5 % decrease of mitochondrial activity indicating the presence of an effect that, however, was still above the tolerability threshold.

Overall, these data suggest that for TrkA activation experiments the concentrations of Erdosteine and Met-1 should be respectively limited to 20 and 50 mM or less when exposing the cells for 90 min, and to 10 mM for both when the exposure time is 24 hrs.

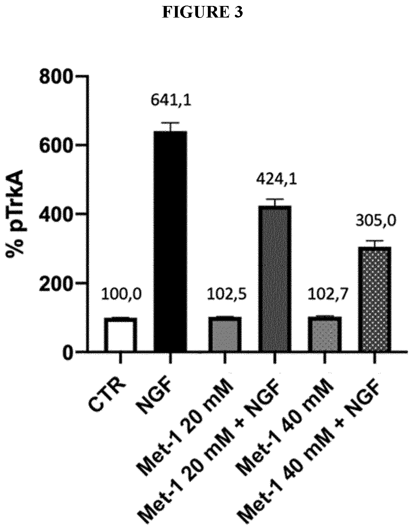

### 2. Effects of Met-1 and erdosteine on the activation of TrkA by NGF (Figures 3-6).

First, the effect of Met-1 exposure for 90 min was tested at the concentrations of 20 and 40 mM on the increase of phospho-TrkA (pTrkA) through the ELISA kit. In this experiment Met-1 concentration was kept below the 50 mM limit.

Cells were exposed for 90 minutes to the medium containing no added substances (the first two columns of figure 3), Met-1 20 and 40 mM (the next four columns of figure 3). Then the medium was changed and either control medium or medium containing NGF 100 ng/ml was added. Data are expressed as % of basal activity in absence of added substances (CTR column). Bars over the columns represent the standard error of independent samples (n=3 or higher). The activation of TrkA was significantly inhibited by both concentrations of Met-1.

### Statistics: Tukey's multiple comparisons test

| Test | Statistically significant | Adjusted P Value | |
|---|---|---|---|
| CTR vs. NGF | Yes | **** | <0.0001 |
| NGF vs. Met-1 20 mM + NGF | Yes | **** | <0.0001 |
| NGF vs. Met-1 40 mM + NGF | Yes | **** | <0.0001 |

As it may be appreciated from figure 3, NGF (100 ng/ml) increased by several folds the amount of pTrkA. Met-1 alone at these concentrations for this time of exposure did not alter the basal pTrkA levels. Met-1 both 20 and 40 mM prevented the activation of TrkA by NGF as indicated by the reduced values of pTrkA when to the cells pre-exposed for 90 min to Met-1 was added fresh medium containing NGF. The inhibition in the case of Met-1 20 and 40 mM was respectively 34 and 52% in a concentration dependent manner.

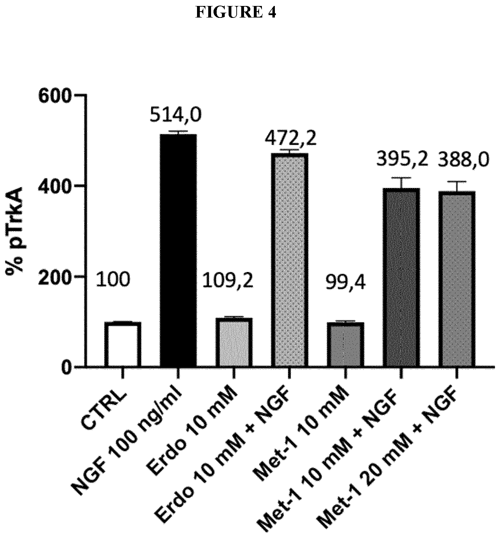

The bar graph in figure 4 reports the effect of erdosteine 10 mM and of Met-1 10 and 20 mM on TrkA activation by NGF. Cells were exposed for 90 minutes to the medium containing no added substances (the first two columns), erdosteine 10 mM (the next two columns), Met-1 10 mM (the two next columns), Met-1 20 mM (the last column). Then the medium was changed and either control medium or medium containing NGF 100 ng/ml was added. Data are expressed as % of basal activity in absence of added substances (CTR column). Bars over the columns represent the standard error of independent samples (n=3 or higher).

### Statistics: Tukey's multiple comparisons test

| Test | Statistically significant | Adjusted P Value | |
|---|---|---|---|
| CTRL vs. NGF 100 ng/ml | Yes | **** | <0.0001 |
| NGF 100 ng/ml vs. Erdo 10 mM + NGF | No | ns | 0.3232 |
| NGF 100 ng/ml vs. Met-1 10 mM + NGF | Yes | *** | 0.0001 |
| NGF 100 ng/ml vs. Met-1 20 mM + NGF | Yes | **** | <0.0001 |

The inhibitory effect of Met-1 confirmed and extended the previous experiment: the inhibition of NGF at 10 and 20 mM was 23 and 24.5% respectively Erdosteine at 10 mM had a small inhibitory effect (8.2% reduction of the response to NGF) which, however, did not reach in this experiment the statistical significance. Both compounds in absence of NGF do not modify the basal activity.

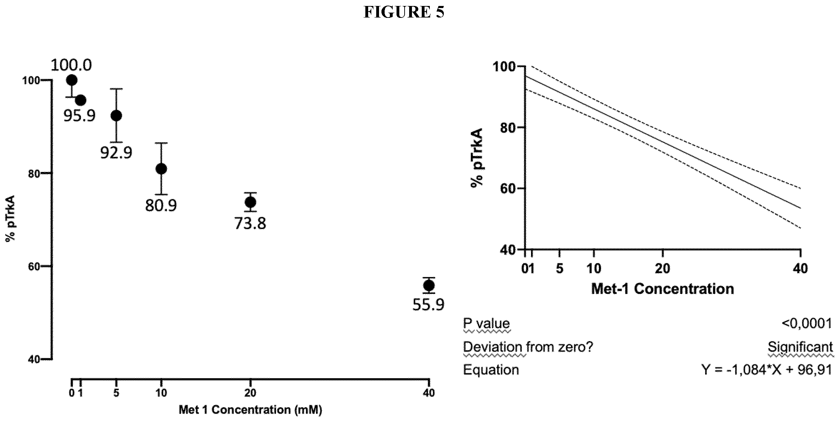

Figure 5 reports for Met-1 the values observed in an *ad hoc* experiment using several Met-1 concentrations, pooled with the data from the previous ones. Cells were exposed to the medium containing no added substances or Met-1 at various concentrations for 90 minutes. Then the medium was changed and a medium containing NGF 100 ng/ml was added. Data are expressed as % of the activity in response to NGF in absence of added substances (the NGF alone or 0 point). Vertical bars represent the standard error of independent samples (n=3 or higher), if not indicated the bars lie within the symbol dimensions. The inhibition of TrkA activation by NGF in presence of Met-1 increasing concentrations within the interval explored at this time (90 min) was linear (insert on the right).

The data were calculated and expressed as percentage of the response to NGF in absence of added compounds. More refined curves based on the percentage of inhibition of phosphorylated TrkA quote due to NGF addition (i.e. isolating the quote over basal rather than calculating the overall activity) make even more evident the effect of the two compounds (not shown). The inhibition is in any case evident and the trend clear even if the proper statistical significance is observed starting with the 10 mM concentration.

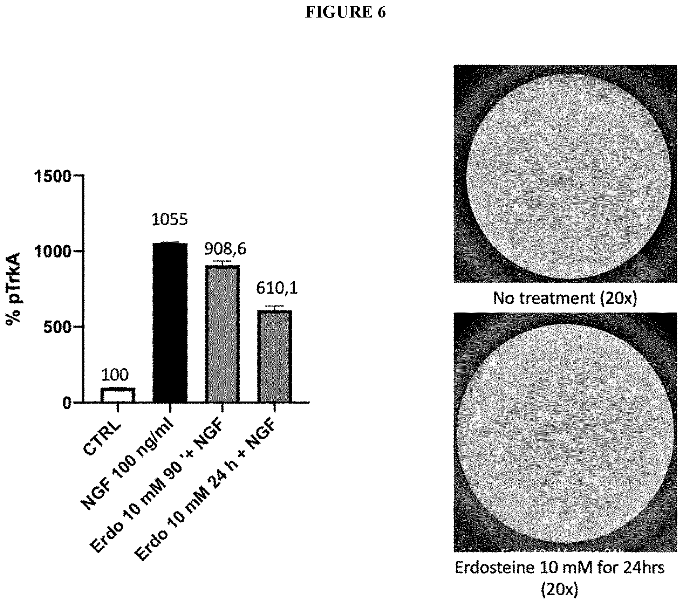

Figure 6 reports the data of an experiment evaluating exposure to erdosteine 10 mM for 24 hrs on cell viability and on the activation of TrkA by NGF. Cells were exposed for 90 minutes or 24 hours to the medium containing no added substances or 10mM erdosteine. Then the medium was changed and a medium containing NGF 100 ng/ml was added. Data are expressed as % of the activity in absence of added substances. Vertical bars represent the standard error of independent samples (n=3 or higher). The two pictures on the right document confirm that erdosteine at 24 hrs does not exert toxic effects on SH-SY5Y cells as also shown by the MTT data in figure 2.

### Statistics: Tukey's multiple comparisons test

| Test | Statistically significant | Adjusted P Value | |
|---|---|---|---|
| NGF 100 ng/ml vs. CTRL | Yes | **** | <0.0001 |
| NGF 100 ng/ml vs. Erdo 10 mM 90 '+ NGF | Yes | * | 0.0324 |
| NGF 100 ng/ml vs. Erdo 10 mM 24 h + NGF | Yes | *** | 0.0001 |

Erdosteine at this concentration did not show important effects on cell viability as shown in figure 2 and by the photo (20x magnification) on the right, documenting the absence of evident cell toxicity after the treatment. In particular, the effect of erdosteine 10 mM on TrkA activation was tested in the same experiment following exposure to the compound for 90 min and 24 hrs. A small inhibitory effect of erdosteine 10 mM after 90 minutes of exposure was confirmed (the inhibition, 13.9%, reached the statistical significance, possibly because of the particularly high response to NGF in this experiment, see also figure 4) and in addition a strong decrease (42.2% inhibition. p<0,0001) of TrkA phosphorylation was observed following 24 hours of exposure. These findings highlight the capability of erdosteine at 10 mM to counteract NGF action and suggest that a longer exposure to the compound increases its ability to impair TrkA activation by NGF.

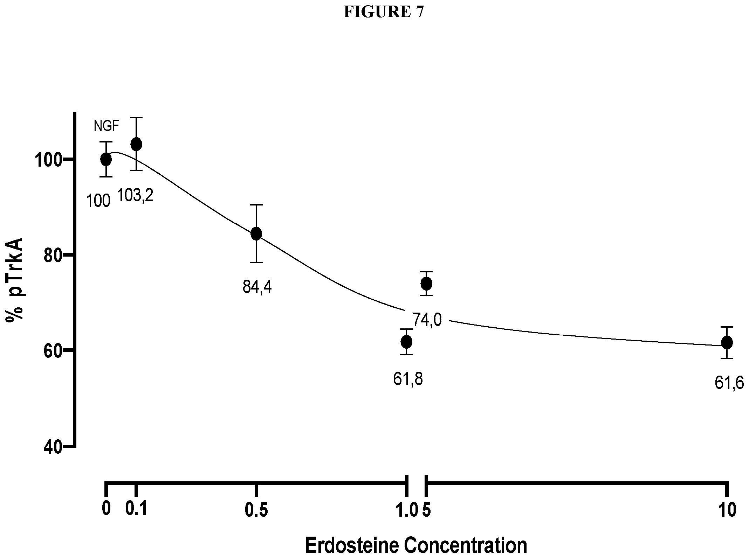

Two follow-up experiments exploring the effect of lower erdosteine concentrations (5.0, 1.0, 0.5, 0.1 mM) were carried out. The results are summarized in figure 7. Cells were exposed for 24 hours to the medium containing no added substances or erdosteine (0.1-10 mM). Then the medium was changed and a medium containing NGF 100 ng/ml was added. Data are expressed as % of the activity in absence of added substances. Vertical bars represent the standard error of independent samples (n=3 or higher). Two experiments combining exposure to erdosteine respectively 0.1, 0.5, 1.0 and 1.0, 5.0, 10.0 mM were combined. The inhibition of TrkA activation by NGF in presence of increasing concentrations of erdosteine within the interval explored at this time (24 hrs) was linear between 0.1 and 1.0 mM then plateaued (curve hand drawn).

### Statistics: Tukey's multiple comparisons test

| Test | Statistically significant | Adjusted P Value | |
|---|---|---|---|
| NGF 100 ng/ml vs. Erdo 0.1 mM 24 h + NGF | No | ns | 0.976 |
| NGF 100 ng/ml vs. Erdo 0.5 mM 24 h + NGF | Yes | * | <0.049 |
| NGF 100 ng/ml vs. Erdo 1 mM 24 h + NGF | Yes | *** | <0.0001 |
| NGF 100 ng/ml vs. Erdo 5 mM 24 h + NGF | Yes | *** | <0.0001 |
| NGF 100 ng/ml vs. Erdo 10 mM 24 h + NGF | Yes | *** | <0.0001 |

As it may be appreciated from the figure, at this time of exposure erdosteine has a significant inhibitory action already at the 1.0 mM concentration and a trend which is appreciable but not strongly statistically significant at the 0.5 mM concentration. The 0.1 mM concentration was ineffective. At the 1 mM concentration the effect already reaches a plateau and further increasing the concentration does not increase the inhibition which levels around 40%.

The data presented confirm that erdosteine and Met-1 are able to inhibit TrkA activation by NGF. The experiments also suggest that the effect is both time and concentration dependent, longer times of exposure (within the timeframe explored) driving higher inhibition rates. This pharmacological property may be of importance given the increasing relevance attributed to potential clinical applications of TrkA inhibition (Chang DS. et al., 2016; Yan W. et al., 2019).

### 3. Effect of Erdosteine and Erdosteine enantiomers on the activation of TRKA by NGF

We examined the effect of the tested compounds by exposing the cells to the test substances for 24 hrs at various concentrations.

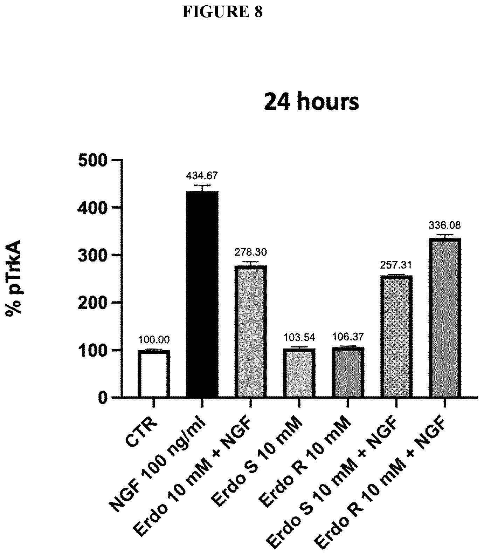

Figure 8 reports the effects of erdosteine, its R and S isomers on TrkA activation by NGF after 24 hrs of exposure to the test substances. The R and S isomers had no effect on the basal activity. The reference substance, erdosteine, inhibited TrkA activation by 36%, the S enantiomer by 41% and the R isomer by 30%.

### Statistics: Dunnett's multiple comparisons test

| Test | Statistically significant | Adjusted P Value | |
|---|---|---|---|
| NGF 100 ng/ml vs. CTR | Yes | **** | <0,0001 |
| NGF 100 ng/ml vs. Erdo 10 mM + NGF | Yes | **** | <0,0001 |
| NGF 100 ng/ml vs. Erdo S 10 mM + NGF | Yes | **** | <0,0001 |
| NGF 100 ng/ml vs. Erdo R 10 mM + NGF | Yes | **** | <0,0001 |

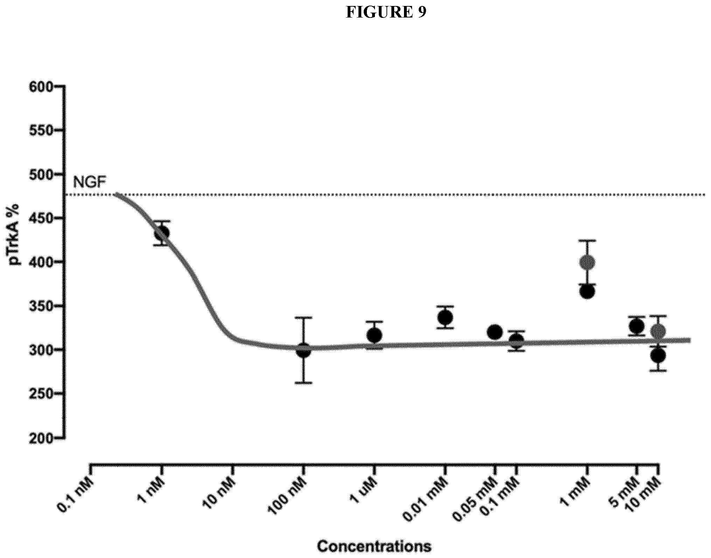

Subsequently, in order to find the inactive concentration of isomer S, we finally performed one experiment in which we treated the cells with the S erdosteine isomer from 1 µM down to 1 nM compared to racemic erdosteine at 10- and 1-mM concentrations (green dots in figure 9). In Figure 9, we pooled all the experiments with isomers S and racemic erdosteine.

### Statistics: Dunnett's multiple comparisons test

| Test | Statistically significant | Adjusted P Value | |
|---|---|---|---|
| NGF 100 ng/ml vs. CTR | Yes | **** | <0,0001 |
| NGF 100 ng/ml vs. Erdo 10 mM + NGF | Yes | *** | 0,0002 |
| NGF 100 ng/ml vs. Erdo 1 mM + NGF | No | ns | 0,8949 |
| NGF 100 ng/ml vs. Erdo S 10 mM + NGF | Yes | **** | <0,0001 |
| NGF 100 ng/ml vs. Erdo S 5mM + NGF | Yes | * | 0,0233 |
| NGF 100 ng/ml vs. Erdo S 1 mM + NGF | No | ns | 0,3069 |
| NGF 100 ng/ml vs. Erdo S 0.1 mM + NGF | Yes | **** | <0,0001 |
| NGF 100 ng/ml vs. Erdo S 0.05 mM + NGF | Yes | * | 0,0134 |
| NGF 100 ng/ml vs. Erdo S 0.01 mM + NGF | Yes | ** | 0,0027 |
| NGF 100 ng/ml vs. Erdo S 1 uM + NGF | Yes | *** | 0,0002 |
| NGF 100 ng/ml vs. Erdo S 100nM + NGF | Yes | **** | <0,0001 |
| NGF 100 ng/ml vs. Erdo S 1 nM + NGF | No | ns | 0,9996 |

The reported data allow to conclude that erdosteine isomers are able to counteract TrkA activation by NGF as does erdosteine. In particular, the S-erdosteine enantiomer had an effect that was comparable as extent of inhibition to the one observed with erdosteine. It is not possible yet a direct comparison of the potency of the S and R enantiomers, even if in the experiment reported in figure 8, directly comparing the activity of the two enantiomers at the same concentration (10 mM), the S one appeared to exert a greater inhibition. In any case the two enantiomers are both capable to inhibit TrkA activation by NGF and do not show additive activity. It also appears that the maximal extent of inhibition has a limit and that in the case of the S-erdosteine isomer there is an effect which is already maximal with 100 nM S erdosteine and does not change even by increasing the amount up to 10 mM, while is null with 1 nM S-erdosteine, preliminarily indicating that the IC50 can be estimated in the 1-100 nM range.

Interestingly racemic erdosteine IC50 can be estimated, from the experiment reported in figure 9, between 0.5 and 1 mM. The action of S-erdosteine in inhibiting TrkA activation by NGF is rather interesting and occurs at concentrations that allow to hypothesize that the compound could be representative of a new class of NGF inhibitors.

### References

- Aydede M., Shriver A., Pain. 2018 Jun;159(6):1176-1177.
- Chang DS. et al., J Pain Res. 2016 Jun 8;9:373-83.
- Denk F. et al., Annual Rev Neurosci. 2017 Jul 25;40:307-325.
- Hirose M. et al., Pain Pract. 2016 Feb; 16(2): 175-82.
- Kamata H. et al., Arch Biochem Biophys. 2005 Feb 1;434(1):16-25.
- Khan N., Smith TM., Molecules. 2015 Jun 9;20(6):10657-88.
- Marchesi N. et al., PLoS One. 2020 Nov 30; 15(11):e0242627.
- Marsh HN. et al., J Cell Biol. 2003 Dec 8;163(5):999-1010.
- Oo WM., Hunter JD., BioDrugs. 2021 Nov;35(6):611-641.
- Pandre MK. et al., Anal Biochem. 2018 Mar 15;545:78-83.
- Pergolizzi JV Jr. et al., Expert Opin Pharmacother. 2021 Aug 30;23:1, 105-116
- Srivastava GK. et al., Sci Rep. 2018 Jan 23; 8(1):1425.
- Testa GM. et al., Pharmacol Res. 2021 Jul;169:105662.
- Yan W. et al., J Med Chem. 2020 Sep 10;63(17):10089.

## Claims

1. Erdosteine, salts, enantiomers thereof or its metabolite of formula 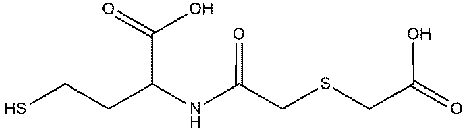 for use in the treatment of neuropathic and nociplastic pain disease.

2. The S-enantiomer of erdosteine for use according to claim 1.

3. The R-enantiomer of erdosteine for use according to claim 1.

## Patentansprüche

1. Erdostein, seine Salze, Enantiomere oder sein Metabolit der Formel 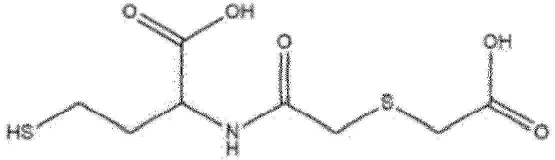 zur Verwendung bei der Behandlung von neuropathischen und noziplastischen Schmerzerkrankungen.

2. S-Enantiomer von Erdostein zur Verwendung nach Anspruch 1.

3. R-Enantiomer von Erdostein zur Verwendung nach Anspruch 1.

## Revendications

1. Erdostéine, sels, énantiomères de ceux-ci ou son métabolite de formule 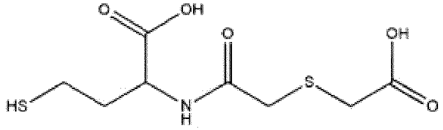 pour une utilisation dans le traitement de la douleur neuropathique et nociplastique.

2. Enantiomère S de l'erdostéine pour utilisation selon la revendication 1.

3. Enantiomère R de l'erdostéine pour utilisation selon la revendication 1.
